# EUROPEAN PATENT SPECIFICATION

(11) **EP 0 962 186 B1**
(45) Date of publication and mention of the grant of the patent: **16.04.2003**
(21) Application number: 99110121.3
(22) Date of filing: 25.05.1999
(51) Int. Cl.: A61B 5/00

(54) **Automated diagnostic system implementing immunoassays and clinical chemistry assays according to a reflex algorithm**
Automatisches Diagnosesystem zur Durchführung von Immunoassays und klinisch-chemischen Assays nach einem Reflexalgorithmus
Système de diagnostic automatisé pour mettre en oeuvre des dosages immunologiques et des dosages de chimie-cliniques selon un algorithme réflexe

(30) Priority: 02.06.1998 US 87871
(43) Date of publication of application: 08.12.1999
(73) Proprietor: Bayer Corporation, Pittsburgh, PA 15205 (US)
(72) Inventor: Wagner, Gerald, Upper Grandview, New York 10960 (US)
(74) Representative: Burkert, Frank

(56) References cited:
- WO-A-97/09678
- WO-A-98/00697
- US-A- 5 316 726
- LINDAHL ET AL.: "Early diagnosis and exclusion of acute myocardial infarction using biochemical monitoring" CORONARY ARTERY DISEASE, vol. 6, 1995, pages 321-328, XP002115419

## Description

### FIELD OF THE INVENTION

The present invention relates generally to diagnostic methods and systems, and more particularly, to an automated diagnostic platform which integrates immunoassays and clinical chemistry assays implemented according to a Reflex algorithm, such as a reflex algorithm for use in the early diagnostic of acute myocardial infarction as also provided by the present invention, which assists in determining the appropriate biochemical tests to be conducted on a given patient and alleviates unnecessary assays.

### BACKGROUND OF THE INVENTION

Essential for the appropriate and optimal treatment and handling of patients who present with suspected myocardial infarction is an early diagnosis within the first hours after the onset of symptoms, e.g., chest pain, that are believed to be of cardiac origin. Although the 12-lead electrocardiogram (ECG) is usually immediately available, it is nondiagnostic on admission for the vast majority (e.g., up to about 60%) of patients. In cases with typical ST-segment elevations, the diagnostic of acute myocardial infarction (AMI) is straightforward. However, in those patients with a non-diagnostic ECG, a diagnostic according to World Health Organization (WHO) criteria can only be made by also considering the results of biochemical marker tests.

There has been interest in new biochemical markers for an early rule-in and rule-out of AMI. The diagnostic sensitivity and specificity of such markers may be high; however, they are often not sensitive enough for confirming or ruling out AMI immediately upon admission. It is a goal in the art to be able to identify at an early stage those patients, among the heterogeneous group of patients with chest pain, who have actually suffered an AMI, for hospital admission and early treatment, while ruling out the possibility of AMI in patients with non-AMI related chest pain to have them appropriately treated and discharged early from the emergency room.

To achieve some of the goals in the art, several methods have been proposed, such as diagnostic algorithms based on clinical data (T.H. Lee et al., 1991, N. Eng. J. Med., 324:1239-1246: J. Jonsbu et al., 1993, Eur. Heart J., 14:441-446) or biochemical markers (P.O. Collinson et al., 1988, Ann. Clin. Biochem., 25:376-382; B. Lindahl et al., 1995, Coron. Artery Dis., 6:321-328). In addition, artificial neural networks, based on clinical data including ECG (W.G. Baxt, 1991, Ann. Intern. Med., 115:843-848; W. G. Baxt, 1992, Ann. Emerg. Med., 21:1439-1444), biochemical markers (J.W. Furlong et al., 1991, Am. J. Clin. Pathol., 96:143-141; S. Pedersen et al., 1996, Clin. Chem 42:613-617) and frequent sampling and measurement of selected markers of AMI (J. Ellenius et al., 1997, Clin. Chem., 43:1919-1925) have been used as an alternative technique in AMI diagnosis.

Reflex algorithms (i.e., algorithms which specify selections of subsequent tests based on results of previous tests, without the need for subjective human decision-making in selecting tests) have been employed in the areas of clinical chemistry and laboratory medicine for more than a decade. Initially, such algorithms were suggested for the assessment of thyroid disease, where an assay for thyroid stimulating hormone (TSH) was the first-line test, and further markers were chosen, or omitted, on the basis of the TSH result (J. Klee, 1987, J. Clin Endocrinol., 64;641-671). To date, thyroid testing is the only area in laboratory medicine in which reflex testing has found acceptance among practitioners in the art. Reflex algorithms for the detection of other disease states and medical conditions are not conventionally employed. Thus, reflex testing and the use of algorithms for the diagnosis of other diseases are needed in the art and promise to improve the diagnostic process through their efficiency, effectiveness and reduction of costs of further unwarranted testing.

WO 98/00697 discloses a high throughput automated immunoassay analyzer instrument, which can perform high volume testing on a broad range of analytes while selecting from among a diverse set of immunoassays for any given sample.

The immunoassay analyzer instrument produces reportable assay results through the processing of specimens and various other components of the chemistry system. This processing involves control and timing of various internal operations as well as the acquisition in processing of data generated internally or through interaction with an external computer system. The immunoassay analyzer instrument is an integrated electromechanical apparatus which processes specimens in order to generate test results. It is comprised of all the mechanical hardware, electronic hardware and software required to perform immunoassays. Many different constituents in the sample can be tested by immunoassay by the analyzer depending on the selection of the biomaterial bound to inert support in the assay tube. The automated design allows reduced user interface (e.g., tests are performed automatically from computer input) including the ability to order, perform and re-assay tests reflexively based on test results without operator intervention.

An abstract by T.E. Caragher et al. (1997, Clin. Chem., 43:S108) discloses serial measurements of several AMI markers at defined time points. The criteria for confirmation of AMI were defined based on the results of the testing algorithm. The algorithm of Caragher et al. is not a reflex algorithm because subsequently ordered tests do not depend on the previous results obtained.

J. Ellenius et al. disclosed a computer assisted approach to diagnose acute myocardial infarctions. In this report, creatine kinase isoforms, myoglobin and Troponin T were measured in short time intervals and the diagnosis was made by processing the results using a neural network. Such a neural network is not performed in the manner of reflex testing and does not try to minimize the number of necessary tests. Instead, it focuses on obtaining the most accurate information as early as possible from several different tests run in very short intervals.

It is appreciated, therefore, that there is a need for further improvements and developments in detecting myocardial infarction, and more particularly, for a reflex method for specific and sensitive detection of AMI, and for an automated diagnostic platform which integrates immunoassays and clinical chemistry assays implemented according to a Reflex algorithm, such as an AMI reflex algorithm.

### SUMMARY OF THE INVENTION

The present invention overcomes the above, and other, limitations of the prior art and the background art by providing a reflex method and system for supporting diagnosis of diseases with biochemical markers. In accordance with one embodiment the present invention is applied to the detecting of myocardial infarction in an individual. The method and system of the present invention comprises a hierarchical ordering of biochemical marker measurement steps in which subsequent biochemical marker measurements are selectively performed based on the results of previous biochemical marker measurements and without the need for human decision making. In accordance with an illustrative implementation according to one embodiment of the present invention, a method for detecting myocardial infarction in an individual includes performing one of a plurality of sequences of biochemical marker measurement steps prescribed by a decision tree, each of the biochemical marker measurement steps including measuring a concentration level of at least one biochemical marker of myocardial infarction in a serum, plasma or whole blood sample obtained from the individual at one of a plurality of times from admission. Each sequence of the decision tree begins with a common first biochemical marker measurement step conducted on a first serum, plasma or whole blood sample obtained from the individual within a first predetermined time from admission. Each of the biochemical marker measurement steps subsequent to the common first step is selectively performed based on results from a precedent biochemical marker measurement step, each sequence terminating in a respective final biochemical marker measurement step conducted on serum, plasma, or whole blood sampled from the individual at one of a plurality of different times subsequent to admission. In addition to other clinical data, an indication of myocardial infarction is provided for the individual based on the sequence of biochemical marker measurement steps performed and on the results of the final biochemical marker measurement step.

In accordance with another aspect of one embodiment of the present invention, an illustrative AMI detection reflex algorithm, which establishes useful biochemical tests for patients with suspected myocardial infarction, begins with the step of testing myoglobin and total creatine kinase activity (total-CK) upon admission to the emergency room or within a short defined time interval after admission. Then, in accordance with the reflex algorithm, if a negative result is obtained for either of these two biochemical marker tests, the two biochemical marker tests are repeated approximately every four hours until there is a positive result, or until the test combination is run a predetermined number (e.g., four) of times. Should the test results always be negative, the testing cascade is finished with a Troponin I test because of its higher specificity and wider time window coverage: a positive result indicates that the patient should be treated for angina and minor myocardial damage; a negative result indicates that causes of chest pain unrelated to cardiac involvement should be considered. If there are any elevated values obtained for myoglobin and/or total -CK during the testing regimen described above, a creatine kinase MB (CKMB) mass test is performed. In the case of a positive result for both the CKMB mass test and a calculated relative index (RI%) of CKMB to total CK, no further testing is required, and an indication of acute AMI according to the World Health Organization criteria may be established. In the case of a negative other than positive CKMB and positive RI%, the sequences specified according to the decision tree end with a Troponin I test as the final test. The cut-offs of the different markers used in the Reflex algorithm are not necessarily identical with the clinically-determined normal ranges and may be adjusted for optimal performance of the Reflex algorithm. The Reflex algorithm is preferably implemented on a computer system, including automated diagnostic systems, and may also be implemented as a computer program stored on a computer-readable medium. In accordance with yet another aspect of the present invention, in addition to determining the next most appropriate biochemical marker test, the Reflex algorithm of the present invention also serves as an Expert System by offering suggestion for clinically explaining the test results and/or recommendations for treatment and/or testing other than the specific sequence of biochemical marker tests needed to detect MI.

In accordance with a further aspect of the present invention, a system is provided as defined in claim 1 and which includes an immunoassay analyzer, a clinical chemistry analyzer, and a processor coupled to the immunoassay analyzer and clinical chemistry analyzer to execute measurements specified by a program executed by the processor in order to facilitate diagnosis of a pathology according to a reflex algorithm which includes at least one immunoassay and at least one clinical chemistry assay.

The method and apparatus of one embodiment of the present invention, by providing a reflex algorithm for detecting AMI, facilities unambiguous and early diagnosis of acute myocardial infarctions. Additionally, it not only aids in the determination of the appropriate biochemical tests that need to be run on a patient who presents chest pain or a suspected heart-associated condition but also, omits the execution of unnecessary assays while ensuring that all necessary combinations of laboratory results are covered. The reflex algorithm of the present invention, by selecting subsequent tests based upon the results of previously-run assays; automatically selects the appropriate biochemical markers for a given clinical situation which concomitantly eliminates the need for human decision-making in selecting the tests, and minimizes the number of necessary tests that have to be run, thus leading to a faster and more reliable diagnosis of AMI. Such features are tantamount to diagnostic efficiency and cost effectiveness.

### BRIEF DESCRIPTION OF THE DRAWINGS

Additional aspects, features, and advantages of the invention will be understood and will become more readily apparent when the invention is considered in the light of the following description made in conjunction with the accompanying drawings, wherein:
FIGURES 1A -1F schematically depict a decision tree of an illustrative Reflex algorithm for detecting myocardial infarction in accordance with an embodiment of the present invention;
**FIG. 2** schematically depicts a functional block diagram of an illustrative computer system used to implement a Reflex algorithm for detecting myocardial infarction in accordance with an embodiment of the present invention; and
**FIG. 3** shows a functional block diagram of an illustrative integrated system for implementing immunoassays and clinical chemistry assays according to a Reflex algorithm, in accordance with an embodiment of the present invention.

### DETAILED DESCRIPTION OF THE INVENTION

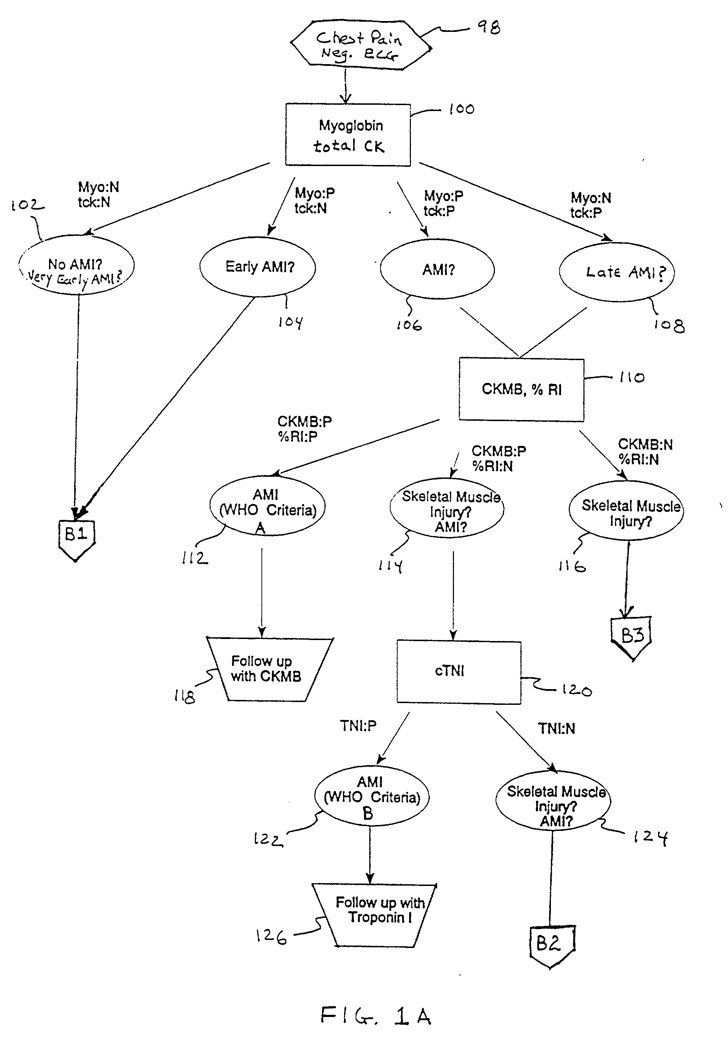

Referring generally to **FIGS. 1A-1F,** there is shown an operational flow or state diagram for a Reflex algorithm in accordance with an embodiment of the present invention. The depicted Reflex algorithm represents a decision tree, or hierarchical organization, of biochemical marker measurements, including both immunochemistry and clinical chemistry assays in which the markers have different appearance kinetics influencing their sensitivities and specificities within different time windows. More specifically, the illustrative Reflex algorithm of **FIGS. 1A-1F** employs myoglobin, total creatine kinase (tCK or total CK) activity, creatine kinase MB (CKMB) mass, and cardiac troponin I (cTNI) biochemical marker measurements. It is known that myoglobin has early sensitivity to myocardial infarction (about 2-3 hours post infarction), while its level returns to normal within about 24 hours, whereas total CK is elevated about 6 to 48 hours post-infarction and peaks about 18 hours after the onset of symptoms. Both myoglobin and especially total CK are relatively inexpensive, but are also non-specific to the myocardium. Troponin I is very specific but appears in the circulation later, and is relatively expensive compared to myoglobin or total CK. The optimum sensitivity for Troponin occurs at about 5-48 hours post-infarction. CKMB has a specificity less than that of Troponin I but greater than that of myoglobin and total CK, and has a sensitivity time window of about 5-48 hours post-infarction.

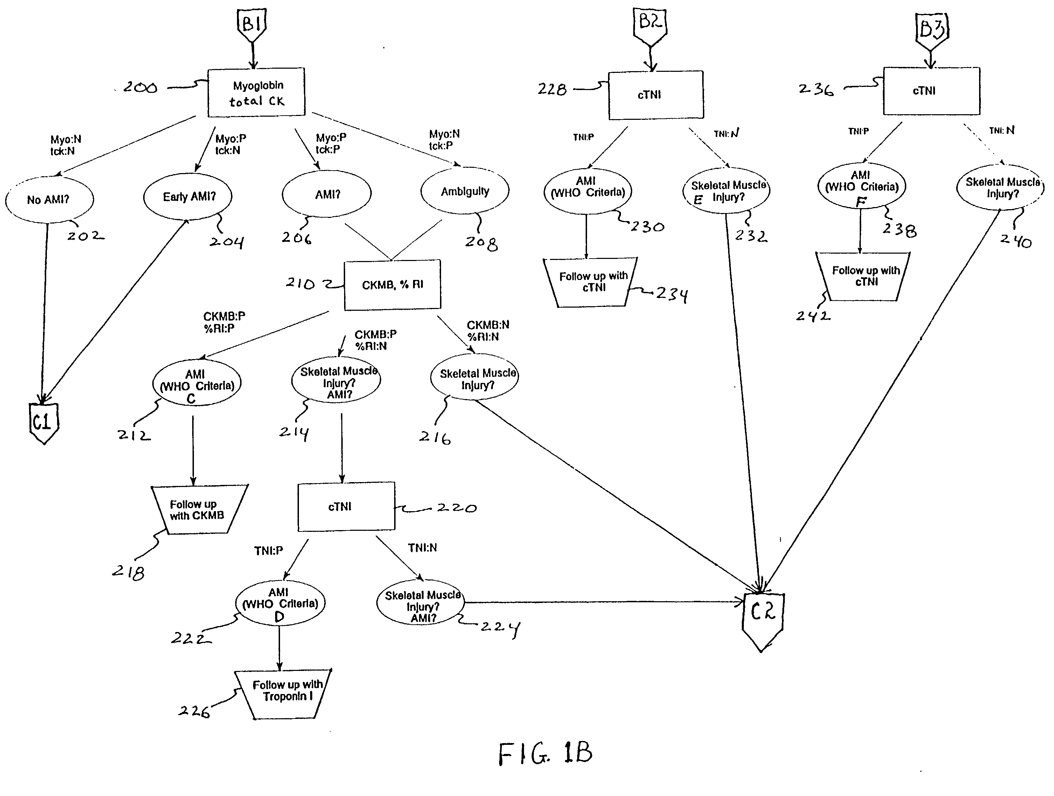

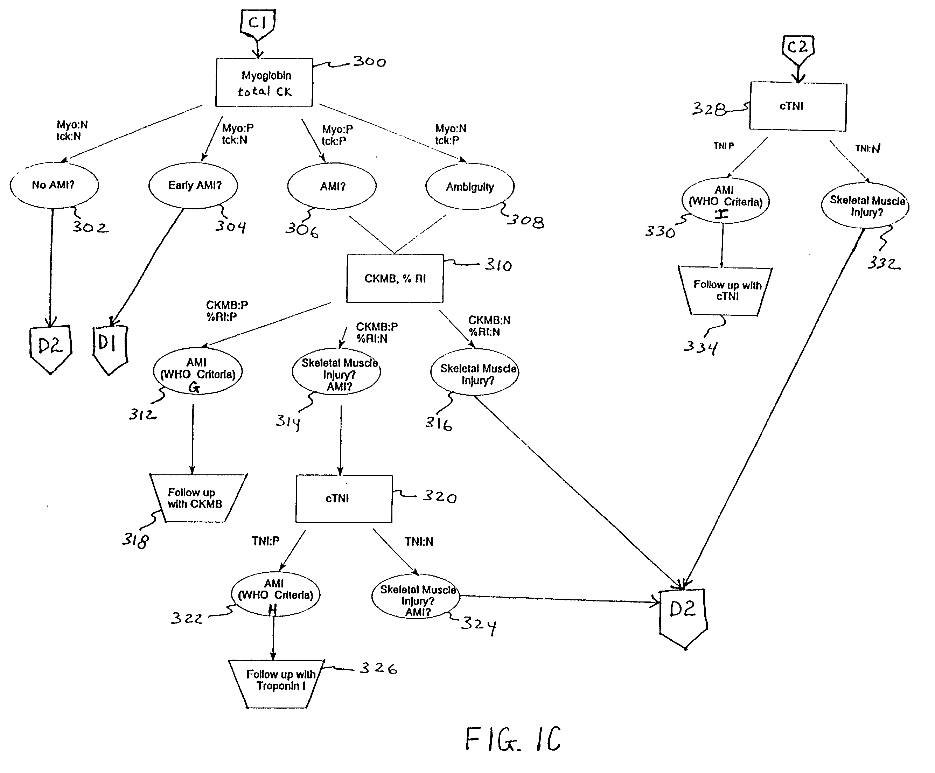

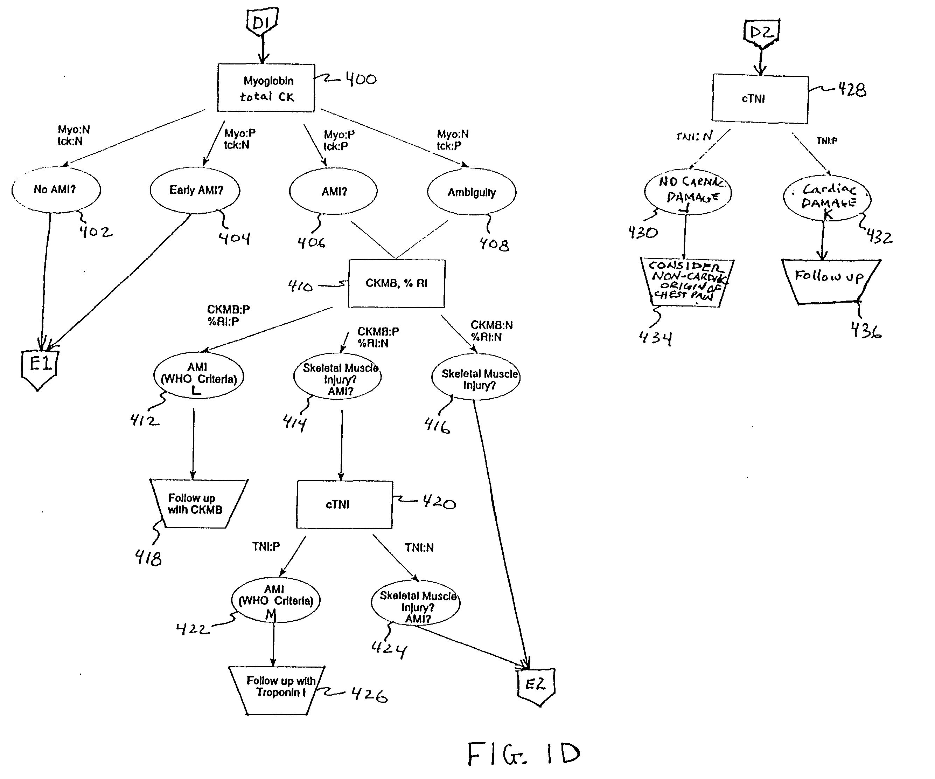

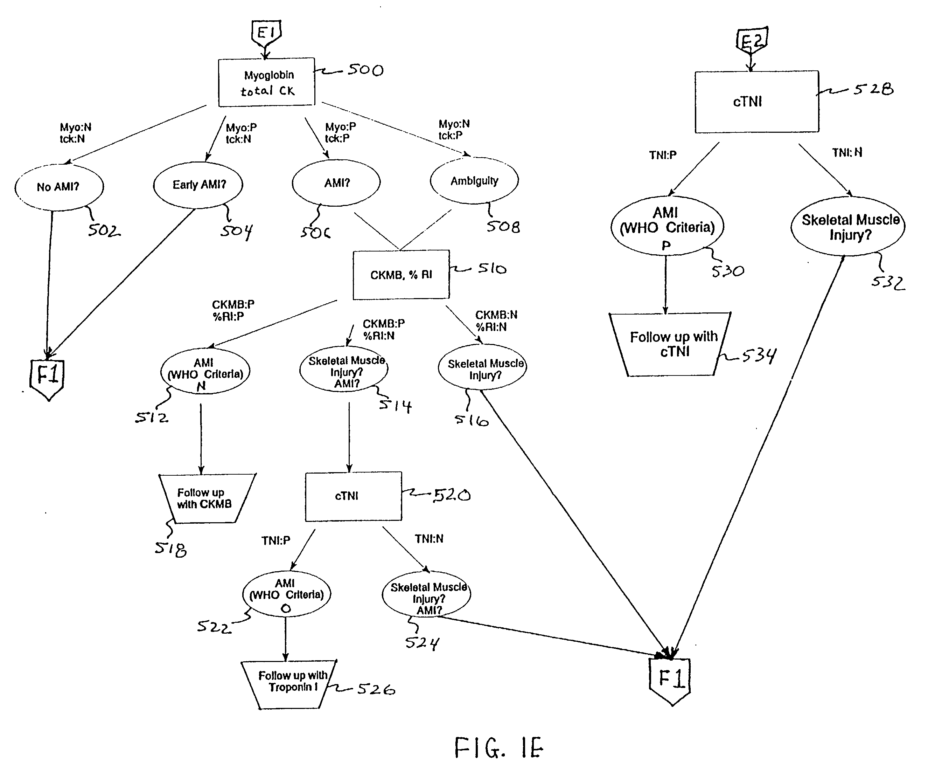

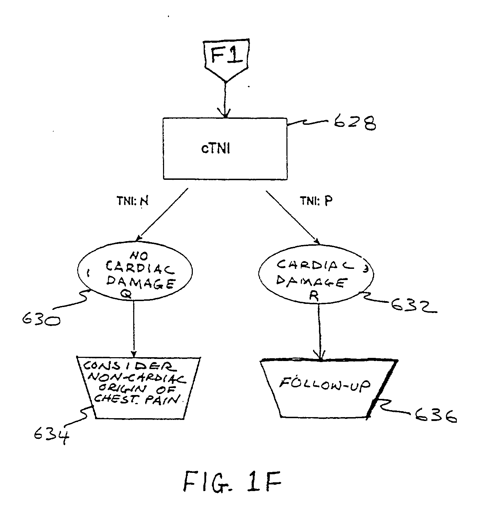

For clarity of exposition, as will be more fully understood hereinbelow, as depicted in **FIGS. 1A-1F**, rectangular shapes indicate assay execution steps (i.e., one or more biochemical marker measurements), elliptical nodes identify provisional or final patient status indications depending respectively on whether or not they are followed by an additional biochemical marker measurement step (i.e., indicated by a rectangle), trapezoidal nodes indicate suggested treatments and/or follow-up procedures based on the diagnostic endpoint (i.e., the final patient status indication) and preferably also on the sequence of foregoing test results which lead to the endpoint. It is noted that the elliptical nodes which are followed by another assay execution step (i.e., the provisional indications) do not represent steps which are necessarily indicated in practicing the Reflex algorithm, but are shown for clarity in describing the underlying logical structure and arrangement of the operational flow. As will be more fully understood below, however, such provisional indications may be provided as suggestions for clinically explaining the test results thus far obtained, and may also be associated with other suggested treatments or recommendations to the physician. It is also noted that all biochemical marker measurements or assays identified in each of **FIG. 1A, FIG. 1B, FIG. 1C, FIG. 1D, FIG. 1E,** and **FIG. 1F** are executed on blood drawn, in order, upon admission (e.g., within about three hours of admission), and in four hour increments subsequent to the time of the initially drawn blood upon admission (i.e., **FIG. 1D** and **FIG. 1E** corresponding to 12 hours and 16 hours post-admission, respectively). In addition, for simplicity, reference to blood, blood sample, or the like being used for a biochemical marker measurement, is generically used to refer to using whole blood, serum, or plasma as appropriate for the assay conducted.

Referring now more specifically to **FIGS. 1A-1D,** upon admitting a patient complaining of chest pain and/or demonstrating an abnormal (i.e., negative) electrocardiogram (ECG) measurement (step 98), both myoglobin and total creatine kinase (total CK) assays are run on blood samples drawn from the patient at that time (step **100**), which is preferably as soon as possible. Initially testing both myoglobin and total-CK is effective in detecting recent (e.g., 2-3 hours post-onset) as well as older (e.g., 5-48 hours post-onset) infarctions.

If in step **100**, the total CK measurement produces a positive result (indicated tcK:P; "positive" total CK meaning that total CK activity is above some threshold level), indicating the possibility of AMI (step **106**) or the possibility of progressed AMI (i.e., late AMI, step **108),** then in step **110** a creatine kinase MB measurement (CKMB) is performed using the first blood sample, and the percentage relative index (%RI) represented by the ratio of CKMB to total CK (using the total CK measurement for the first blood sample) is calculated. If the CKMB concentration is above a threshold level (i.e., positive, indicated as CKMB:P) and %RI is above a threshold (i.e., positive, indicated as %RI:P), then AMI, consonant with the World Health Organization definition, is indicated as the diagnosis of cardiac status for the patient (step **112**), and preferably a follow-up CKMB measurement is suggested to the physician (step **118**). Alternatively, if in step **110** the CKMB concentration is negative and %RI is negative (i.e., CKMB:N, %RI:N), indicating a possible skeletal muscle injury causation (step **116**), then to further titrate the diagnosis and more specifically assess cardiac status, the flow proceeds via step **236** wherein the concentration of cardiac troponin I (cTNI) is measured for blood drawn about four hours after the first blood sample was drawn. If, however, in step **110** the CKMB concentration is positive and %RI is negative (i.e., CKMB:P, %RI:N), indicating a possible AMI or possible skeletal muscle injury causation (step **114**), then cTNI is measured for the first blood sample (step **120**). If in step **120** cTNI is positive, then AMI, consonant with the World Health Organization definition, is indicated as the diagnosis of cardiac status for the patient (step 122), and preferably a follow-up troponin I measurement is suggested to the physician (step **126**). Alternatively, if in step **120** cTNI is negative (i.e., TNI:N), indicating a possible skeletal muscle injury causation (step **124**), then to further titrate the diagnosis and more specifically assess cardiac status, the flow proceeds to step 228 wherein the concentration of troponin I is measured for blood drawn about four hours after the first blood sample was drawn.

It is appreciated that in accordance with the present invention, the illustrative embodiment of **FIG. 1A** provides possible pathways (i.e., a pathway representing a series of biochemical marker measurement steps performed according to the Reflex method, also referred to herein as a thread or sequence) for early detection of AMI as represented by the two diagnostic endpoints indicative of AMI (i.e., steps **112** and **122**) which result from biochemical measurements on blood drawn upon admission only and do not require further blood samples that may be needed to diagnosis cardiac status in other patients according to the Reflex method.

Referring again to step **100**, both tests indicating negative results (indicated as Myo:N, tcK:N; "negative" total CK meaning that total CK activity is below some threshold level; "negative" myoglobin meaning that myoglobin blood concentration is less than some threshold level) suggests that AMI is either not present or in its very early stages (step **102).** Similarly, a positive myoglobin result (i.e., indicating a myoglobin blood concentration greater than a threshold level) and negative total CK result suggests that AMI may be in its early stages (step **104**) because, as described, the myoglobin assay has its greatest sensitivity earlier than that of the total CK assay sensitivity. Since either of these cases in which initially measured total CK is negative may be due to the patient exhibiting early AMI, in either event an additional set of myoglobin and total CK tests are run (step **200**) on blood drawn preferably about four hours after the time that the blood was drawn for the first set of myoglobin/total CK assays.

Referring now to steps **200-226,** it is noted that these steps are directly analogous to hereinabove described steps **100-126**, and thus will not be specifically described for purposes of brevity and clarity of exposition. As mentioned, biochemical marker measurements in steps **200-226** are preferably conducted on blood sampled about four hours after the first blood sample was drawn from the patient, steps **200-226** thus representing a measurement sequence corresponding to steps **100-126** but time delayed in order to identify, diagnose, and/or titrate delayed presentation of AMI relative to time of admission. It is also noted that if either (i) both CKMB and %RI are negative in step **210**, or (ii) cTNI is negative in step **220**, each case indicative of possible skeletal muscle injury (steps **216** and **224**), then to further titrate the diagnosis and more specifically assess cardiac status, the flow proceeds to step **328** wherein the concentration of troponin I (cTNI) is measured for blood drawn about eight hours after the first blood sample was drawn. In addition, in step **200**, similar to step **100**, in either case where total CK is negative (steps **202** and **204**), myoglobin and total CK will be measured on a subsequently drawn blood sample (step **300**), which in this case is for blood drawn preferably about eight hours after the first blood sample (i.e., about four hours after the second blood sample). More specifically, if in step **200** either: (i) both tests indicate negative results (i.e., Myo:N, tcK:N), suggesting that AMI is not present (step **202**) because there was no positive change in total CK (i.e., no increase in activity beyond the threshold) and myoglobin either remained or became negative, or (ii) myoglobin is positive and total CK is negative (Myo:P, tCK:N), suggesting that the patient may be in the early stages of AMI (step **204**) because total CK remained negative and myoglobin remained or became positive, then in step **300** myoglobin and total CK is measured for blood drawn about eight hours after the first blood sample was drawn.

Referring to step **228**, as mentioned, since a cTNI measurement of the first blood sample (i.e., step **120**) did not indicate a level sufficient to indicate AMI, cTNI is measured for blood sampled about four hours after the first blood sample was drawn in order to further titrate a diagnosis. If in step **228** cTNI is positive, then AMI, consonant with the World Health Organization definition, is indicated as the diagnosis of cardiac status for the patient (step **122**), and preferably a follow-up troponin I measurement is suggested to the physician (step **322).** Alternatively, if in step **228** cTNI is negative (i.e., cTNI:N), indicating a possible skeletal muscle injury causation (step **124),** then to still further differentiate a diagnosis and more specifically assess cardiac status with a statistically significant degree of certainty, the flow proceeds to step **328** wherein the concentration of troponin I is measured for blood drawn about eight hours after the first blood sample was drawn.

Referring to step **236**, since for the first blood sample CKMB and %RI were both negative despite a positive cTNI measurement, cTNI is measured for blood sampled about four hours after the first blood sample was drawn in order to further assess the cause of elevated cTNI and to differentiate a diagnosis. If in step **236** cTNI is positive, then AMI is indicated as the diagnosis of cardiac status for the patient (step **238**), and preferably a follow-up troponin I measurement is suggested to the physician (step **242).** Alternatively, if in step **236** cTNI is negative (i.e., cTNI:N), indicating a possible skeletal muscle injury causation (step **240),** then to still further differentiate a diagnosis and more specifically assess cardiac status with a statistically significant degree of certainty, the flow proceeds to step **328** wherein the concentration of troponin I is measured for blood drawn about eight hours after the first blood sample was drawn.

In step **328,** if cTNI is positive, then cardiac damage which is possibly but not likely AMI is indicated as the diagnosis of cardiac status for the patient (step **330**), and preferably a follow-up troponin I measurement is suggested to the physician (step **242**). It is noted that the degree of cardiac damage may be further indicated, as well as different suggested treatments, based on the measured troponin I level (e.g., greater than 0.9 ng/ml indicating AMI rather than unstable angina). Alternatively, if in step **328** cTNI is negative (i.e., cTNI:N), still indicating a possible skeletal muscle injury causation (step **332**), then to still further differentiate a diagnosis and more specifically assess cardiac status with a statistically significant degree of certainty, the flow proceeds to step **428** where the concentration of troponin I is measured for blood drawn about twelve hours after the first blood sample was drawn.

Referring now to step **300**, which is performed if total CK activity was negative for both the first blood sample (step **100**) and the blood sample drawn four hours after the first blood sample (step **200**), if myoglobin and total CK measurements both produce negative results, then it is likely that no AMI is present (step **302**) because the total CK activity never exceeded the threshold and the myoglobin concentration either decreased below threshold or never exceeded the threshold. Accordingly, in order to further differentiate the diagnosis and more specifically assess cardiac status, in step **428** the concentration of troponin I (cTNI) is measured for blood drawn about twelve hours after the first blood sample was drawn.

If, however, in step **300** the total CK measurement produces a positive result, indicating the possibility of AMI (step **306**) or the possibility of progressed AMI (i.e., late AMI, step **308**), then in step **310** a creatine kinase MB measurement (CKMB) is performed using the blood sampled at about eight hours after the first blood sample, and the percentage relative index (%RI) represented by the ratio of CKMB to total CK (using the total CK measurement for the eight-hour blood sample) is calculated. If the CKMB concentration is above a threshold level (i.e., positive, indicated as CKMB:P) and %RI is above a threshold (i.e., indicated as %RI:P), then AMI, consonant with the World Health Organization definition, is indicated as the diagnosis of cardiac status for the patient (step **312**), and preferably a follow-up CKMB measurement is suggested to the physician (step **318).** Alternatively, if in step **310** the CKMB concentration is negative and %RI is negative (i.e., CKMB:N, %RI:N), indicating a possible skeletal muscle injury causation (step **316**), then to further differentiate the diagnosis and more specifically assess cardiac status, the flow proceeds to step **428** wherein the concentration of troponin I is measured for blood drawn about twelve hours after the first blood sample was drawn. If, however, in step **310** the CKMB concentration is positive and %RI is negative (i.e., CKMB:P, %RI:N), indicating a possible AMI or possible skeletal muscle injury causation (step **314**), then cTNI is measured for the blood sample drawn about eight hours after the first blood sample was drawn (step **320**). If in step **320** cTNI is positive, then AMI, consonant with the World Health Organization definition, is indicated as the diagnosis of cardiac status for the patient (step **322**), and preferably a follow-up troponin I measurement is suggested to the physician (step **322**). Alternatively, if in step **320** cTNI is negative (i.e., TNI:N), indicating a possible skeletal muscle injury causation (step **324**), then to further differentiate the diagnosis and more specifically assess cardiac status, the flow proceeds to step **428** wherein the concentration of troponin I is measured for blood drawn about twelve hours after the first blood sample was drawn.

Referring now to step **428**, which, as may be understood from the foregoing, will be performed as a result of any one of multiple pathways unless terminated before, if the troponin I concentration exceeds a threshold, corresponding to a positive result (i.e., TNI:P), then cardiac damage is indicated (step **432**) as a diagnosis, and preferably certain specific follow up procedures are recommended (step **436**) (e.g., follow up visit for troponin I measurement). It is noted that the degree of cardiac damage may be further indicated, as well as different suggested treatments, based on the measured troponin I level (e.g., greater than 0.9 ng/ml indicating AMI rather than unstable angina). If, however, the troponin I measurement result is negative (i.e., cTNI:N), then no cardiac damage is indicated as the diagnosis (step **430),** and it is recommended that the patient be treated for non-cardiac origin of chest pain (step **434).**

Referring again to step **300**, if myoglobin is positive and total CK is negative, indicating a possible early AMI (step **304**), then yet a further myoglobin and total CK measurement is taken, using blood sampled at about twelve hours after the first blood sample was drawn (step **400**). For purposes of brevity and clarity of exposition, the progression of each and every step within steps **400-426,** steps **500-526**, steps **528-534**, and steps **628-636** are not described in detail because they directly correspond, in order, to the flow of steps **200-226**, steps **300-326**, steps **328-334**, and steps **428-436** with the following distinctions. First, for corresponding biochemical marker measurement steps, the blood is drawn about eight hours later (e.g., steps **628-636** and steps **428-436** are performed on blood drawn about 20 hours and 12 hours, respectively, after the first sample was drawn). Second, whereas in step **300** a positive myoglobin result and a negative tCK result are followed by a myoglobin and total CK measurement on a later drawn blood sample in step **400** (i.e., initiating the time delayed pathways represented by steps **400-426,** steps **500-526**, steps **528-534**, and steps **628-636**), in step **500** a positive myoglobin result and a negative total CK are followed by a cTNI measurement, since troponin I has not been elevated (i.e., at a positive level) for measurements on blood sampled every four hours through sixteen hours.

It is understood that the time delayed measurement pathways following a measurement of positive myoglobin and negative tCK in step **300** are provided to ensure the possibility of properly diagnosing AMI in the event that the elevated myoglobin on blood sampled eight hours after the first blood was drawn is due to a delayed presentation of AMI relative to time of admission (i.e., early AMI). It may be further appreciated that since a measurement of positive myoglobin and negative tCK in step **300,** after twelve hours, may be indicative of early AMI detection, in addition to specifying a subsequent biochemical marker measurement step (i.e., step **400),** in accordance with the present invention, not only may this provisional indication of early AMI be noted to a physician (corresponding to noting time of AMI onset) but also a recommendation of certain treatments to mitigate further cardiac damage may also be made to the physician.

More generally, throughout various points in the Reflex algorithm of **FIGS. 1A-1F** provisional indications (i.e., indicated by elliptical shapes), and possibly also certain recommended treatments or other clinical tests, may be noted to a physician as the measurements specified by the Reflex algorithm progressively are performed before an endpoint is reached. In addition, the indications and/or recommended treatments at a given point, including the endpoints, in the Reflex method may be not only dependent on the endpoint itself (i.e., characterized by at least one sequence of tests that result in the endpoint), but also further dependent on the specific pathway traversed (i.e., the specific sequence of tests performed and their results) thereto, since, as depicted, more than one pathway may lead to a common point in the flow. As an example of endpoint-dependent recommendation, an early diagnosis of AMI within the four hour time window may be suggestive of invasive treatment (e.g., enzymatic lysis or rescue PTCA), whereas later diagnosis of AMI (e.g., 12 hours) may be suggestive on non-invasive treatments. By way of example of specific-sequence-dependent diagnosis, consider that the indication and/or recommendation at step **330** preferably depends on whether it was reached via path **110→120→228→328** or path **200→210→328:** the former path indicating AMI as very likely because CKMB was elevated, the latter path indicating a reasonable likelihood of unstable angina because CKMB was not elevated. In this respect, it is appreciated that the Reflex method provides for an Expert system for assessing and treating patients with possible cardiac damage. Such an Expert system may also draw upon additional patient information, such as patient medical history, other clinical tests performed since admission, and family history, preferably stored in a database, in order to provide a diagnosis and/or a treatment recommendation.

It may be appreciated that in accordance with the present invention, the illustrative embodiment of **FIGS. 1A-1F** provides possible pathways for detection of AMI or cardiac damage without requiring that all blood samples included in the overall Reflex method be drawn. More specifically: two diagnostic endpoints are provided based only on blood drawn upon admission (i.e., steps **112** and **122**); four diagnostic endpoints are provided based only on blood drawn upon admission and four hours later (i.e., steps **212, 222, 230,** and **238**); three diagnostic endpoints are provided based only on blood drawn upon admission, and four and eight hours later (i.e., steps **312, 322,** and **330**); four diagnostic endpoints are provided based only on blood drawn upon admission, as well as four, eight, and twelve hours later (i.e., steps **412, 422, 430,** and **432**); three diagnostic endpoints are provided based only on blood drawn upon admission, as well as four, eight, twelve, and sixteen hours later (i.e., steps **512, 522,** and **530);** and only two endpoints (i.e., steps **630** and **632)** are provided based on all blood samples included in the overall Reflex method (i.e., blood drawn every four hours up through twenty hours after the first sample was drawn).

As may be more fully appreciated in view of the foregoing description of **FIGS. 1A-1F**, the design of such a Reflex method for detecting AMI is based on sequencing biochemical marker measurements having various sensitivity, specificity, and appearance kinetics in such a manner so as to include pathways that: (i) result in an indication of no cardiac damage with a high degree of confidence (i.e., small probability of false negative), and (ii) result in an indication of AMI without always requiring that measurements be made on blood sampled for all blood sampling times used in the Reflex method, while accounting for the possibility of various onset times of AMI relative to time of admission. As such, it is understood that many variations and alternative Reflex algorithm implementations are possible. For instance, other markers may be substituted for ones used in the Reflex algorithm of **FIGS. 1A-1F;** for example, glycogen phosphorylase or heart-type fatty acid binding protein may be substituted for myoglobin, or Troponin T may be substituted for Troponin I. In addition, alternative Reflex algorithms may be designed and implemented using any of myriad other biochemical markers having various sensitivity, specificity, appearance kinetics, and costs, including for example: myoglobin, total creatine kinase, creatine kinase MB, troponin T, troponin I, glycogen phosphorylase BB, lactate dehydrogenase, heart-type fatty acid binding protein (h-FABP), carbonic anhydrase III, actin, myosin, and creatine kinase MB isoforms.

In addition, the design of such a Reflex method may also consider other factors, such as the associated monetary cost of the different biochemical marker measurements, time of blood sampling, and assay threshold levels. For instance, in the Reflex algorithm of **FIGS. 1A-1F,** troponin I measurements could replace CKMB measurements if cost were not a consideration; however, since troponin I is relatively expensive, CKMB measurements are implemented in various pathways (e.g., lower risk) to further differentiate a diagnosis such that a troponin I measurement may not be necessary. Generally, the times at which the patient's blood is drawn are selected in accordance with the preferred times (e.g., based on time-dependent sensitivity) for the various assays, as known from established testing procedures. Standard thresholds/levels (e.g., from the published literature) may be implemented for the different assays used; however, as may be further understood below, the thresholds preferably may be adjusted to optimize diagnosis (e.g., a more conservative threshold to minimize false negatives) for a given Reflex algorithm decision tree structure.

Accordingly, it is appreciated that myriad variations of the illustrative Reflex algorithm depicted in **FIGS. 1A-1F** are possible, as well as myriad alternative Reflex algorithm implementations in accordance with the present invention. In any event, once a preliminary Reflex algorithm is designed according to these principles and criteria, its efficacy in classifying cardiac patients may be assessed by clinical testing of patients using each and every assay employed in the algorithm (and perhaps other tests as well), and comparing the classification using the designed Reflex algorithm to the results indicated by all the tests. An important factor is avoiding any false negative results or other mis-classification which would adversely affect patient classification, diagnosis, and treatment. By analyzing these results, it may be understood how the preliminary Reflex algorithm should be, if at all, modified in order to enhance classification (e.g., adding additional testing steps to certain branches of the algorithm and/or adjusting certain threshold levels, such as increasing a threshold level to avoid false negatives) and/or efficiency (e.g., eliminating certain steps of a branch in the algorithm and/or adjusting certain threshold levels). It is noted that for the threshold levels used in the illustrative embodiment represented in **FIGS. 1A-1F,** when total CK is positive it is not possible for CKMB to be negative and %RI to be positive.

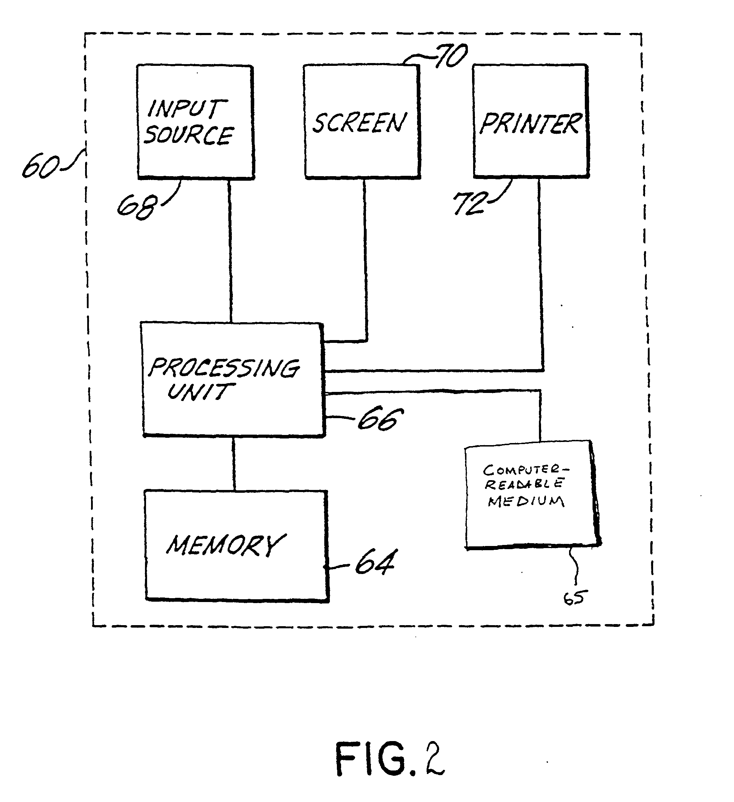

In accordance with an embodiment of the present invention, an AMI Reflex method such as that illustrated in **FIGS. 1A-1F** is preferably implemented programmatically in a processing system. It is appreciated that there are myriad processing system arrangements as well as programming paradigms for implementing the AMI Reflex method. For example, in a relatively simple arrangement, **FIG. 2** illustrates a conventional digital computer system **60,** comprising processing unit **66** coupled to memory **64** (e.g., RAM), another computer-readable medium **65** (e.g., flash memory, magnetic hard-drive, CD-ROM, etc.), an input device **68** (e.g., a keyboard and/or mouse and/or digital data input port), and output devices such as display **70** and printer **72**, and wherein computer system **60** implements an AMI Reflex algorithm (e.g., the algorithm depicted in **FIGS. 1A-1F)** by stored-program execution. For instance, input device **68** implemented as a keyboard may be used by an operator to input results from one or more biochemical marker measurements, the input results then undergoing processing by program control of processing unit **66** in accordance with the AMI Reflex method, and subsequent biochemical marker measurement steps, diagnoses, and/or treatment recommendations specified by the AMI Reflex method according to such processing being output to display **70** and/or printer **72.** It is understood that digital computer system **60** may also have access to a patient database (e.g., stored on computer-readable medium **65**, or via a network to which computer system **60** has access) which includes other information to facilitate diagnosis or treatment.

In somewhat more detail, in such an implementation, for each identifiable patient the processor **66** executes the AMI Reflex algorithm in order to determine one or more biochemical marker measurements to be performed, and prompts an operator (e.g., via display **70**) to execute the determined one or more biochemical marker measurements. After executing the measurements on an immunoassay analyzer (e.g., Immuno I, manufactured by Bayer Corporation) and/or on a clinical chemistry analyzer (e.g., OpeRA, manufactured by Bayer Corporation), the operator inputs the results of the measurements for the patient into the processing system via, for example, the keyboard and/or mouse **68**. In response to such input, the processing system specifies either an additional biochemical marker measurement(s) for execution or an indication of the patient's myocardial status (e.g., a diagnosis when the input is associated with the final biochemical marker measurement(s) of a measurement sequence). In addition, at various points during execution of the Reflex algorithm, the processing system may indicate suggested additional tests or treatments to be conducted and/or suggestions for clinically explaining the test results. More specifically, by way of example, if the early stages of myocardial infarction are provisionally detected (e.g., based on the progression along a certain sequence of biochemical marker tests) before completing a full sequence of biochemical measurements, the processing system may, in addition to specifying subsequent biochemical marker tests to be executed for further titrating cardiac status, recommend certain treatments to mitigate further cardiac damage.

For the system of **FIG. 2**, various data structures or programming paradigms may be used to programmatically implement the AMI Reflex method and user interface. For example, the overall decision tree structure of **FIGS. 1A-1F** may be implemented as a hierarchy of downwardly linked list of records, each record having associated fields which may contain elements relating to provisional or final indications (e.g., indications shown in the ellipses) and/or provisional or final recommended treatments to be output to display **70** and/or printer **72**, as well as pointers to other records in the list. As inputs are received via input device **68,** a main program and/or various subroutines or modules use the inputs to identify an appropriate pointer to point to the next record, and appropriate subroutines or modules handle processing and/or outputting any appropriate information from the fields. In addition, the linked list may be further logically partitioned compared to the illustrative depiction in **FIGS. 1A-1F** such that indications and/or recommended treatments are more specifically dependent on the specific pathway traversed. In this illustrative programmatic implementation, the program and/or linked list data may be stored in memory **64** (e.g., RAM) and processing unit **66** (CPU) processes these signals to effect the AMI Reflex algorithm.

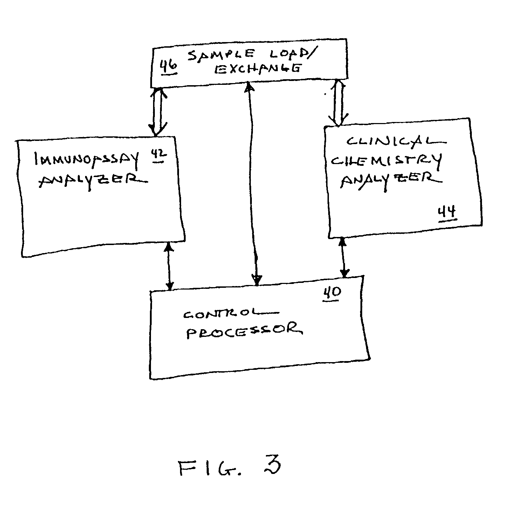

As may be appreciated, in an alternative embodiment depicted in **FIG. 3**, a control processor **40**, such as digital computer system **60** of **FIG. 2** or a microcontroller or microprocessor with any associated elements (e.g., memory, etc.), may be interfaced, by way of well known interfacing techniques, or otherwise integrated, with an immunoassay analyzer **42** and a clinical chemistry analyzer **44** to control and/or acquire data from assay test equipment. For example, immunoassay analyzer **42** may be an Immuno I manufactured by Bayer Corporation, and clinical chemistry analyzer **44** may be an OpeRA manufactured by Bayer Corporation, each interfaced to a personal computer or workstation which executes a program implementing the AMI Reflex algorithm (e.g., using PDC Concentrator software, from Technidata of France). It is understood that in such a system, the clinical chemistry analyzer and the immunoassay analyzer may run different samples (e.g., from different patients) concurrently in response to separate commands (e.g., writing into the respective loadlists of the analyzers) from processing system **40**. Sample transfer between immunoassay analyzer and clinical chemistry analyzer may be implemented by a sample load/exchange system **46**, which may be any of various known sample transfer mechanisms and systems (e.g., Labcell manufactured by Bayer, or LabInterlink manufactured by LabFrame of Omaha, Nebraska), shown under the control of control processor **40**. It is understood that coupling of control processor **40** to sample load/exchange system **46,** immunoassay analyzer **42**, and clinical chemistry analyzer **44** may be implemented in various ways, such as dedicated buses or ports and/or shared buses or ports. Also, immunoassay analyzer **42** and clinical chemistry analyzer **44** may be operative in controlling sample exchange via sample load/exchange system **46**.

It is understood that various processor arrangements may be implemented to provide an immunoassay analyzer and a clinical chemistry analyzer which are coordinately controlled according to an AMI reflex algorithm in accordance with the present invention, such that all tests may be specified and automatically executed without human intervention, other than the ministerial task of drawing additional blood samples that may be required as requested by the processing system implementing the AMI Reflex algorithm. For example, it is understood that the system of **FIG. 3** may be implemented to closely integrate control processor **40** and the analyzers into a single instrument or platform, for example, by dedicating the control processor to the analysis equipment, each analyzer having its own processor. Alternatively, a single processor (e.g., control processor **40**) may directly control both analyzers, with the analyzers not having their own local processors. In yet another alternative implementation, the AMI Reflex algorithm may be executed cooperatively on only two processors, each dedicated to one of the analyzers, with a sample transfer system between the analyzers. In a further alternative implementation, the immunoassay and clinical chemistry analyzers may have their own processors and be networked with one or more control processors or servers via a private network (e.g., a local area network (LAN), or a private wide area network (WAN)), or via a public network (e.g., Internet, WAN, or public-switched telephone network dial-up) to provide a public network-based Reflex algorithm service.

As mentioned, each analyzer may have its own, dedicated (e.g., local) processor, and a third control processor may coordinate overall processing according to the AMI Reflex method for the samples; such an implementation may employ various multiprocessing or parallel processing architectures or paradigms. By way of a simple example, a master/slave implementation may be employed, with the third control processor being the master, the two local processors being slaves. In such an implementation, it is understood that programmatic implementation of the Reflex algorithm may be distributed in various ways between control and each local processor. For example, for a given sample, the control processor may explicitly command each biochemical marker measurement for each analyzer in accordance with the AMI Reflex algorithm, and the local processors each controlling all the steps required to complete the specified measurement (e.g., sample and reagent handling, marker concentration measurement, etc.). Alternatively, the respective local processors may be programmed to execute, in response to a single command or message from the control processor, subroutines or subsequences of the Reflex algorithm which include a series of measurement steps that are of the respective type measured on the analyzer (i.e., clinical chemistry assay or immunoassay), and the control processor may maintain overall coordination between the analyzers for branches between immunoassays and clinical chemistry assays for the various pathways of the AMI Reflex algorithm. As various such branch points are encountered by the local processor (e.g., either at some point within the subroutine as conditioned on the results of biochemical marker measurement(s), or at the end of a subroutine) of the analyzer processing a given sample, the local processor may communicate a message to the control processor (e.g., using an interrupt request) indicative of the branch point, and the control processor can appropriately communicate a message to the other analyzer as to which as to what measurement or subroutine should be executed on the sample (or take other appropriate action, such as provide a final diagnosis and treatment recommendations).

As also mentioned, in an alternative implementation, analyzers and control processors or servers may be coupled over a network. It is understood that in such a system, for example, the AMI Reflex method may be an application implemented by a server, and AMI Reflex method data may also be written into a patient database (e.g., directly from the automatic assay testing equipment). Alternatively, an integrated automatic diagnostic combined immunoassay/clinical chemistry assay analyzer system may be implemented as a node on the network, and have access to other network resources such as patient databases. Such a network implementation is well-suited for implementing an Expert system.

For each of these illustrative implementations, the AMI Reflex algorithm may be implemented according to various programming paradigms or data structures, some of which have been described by way of example for a particular implementation. For example, the program may be generally structured according to a hierarchical downward linked list of files. Also, the Reflex algorithm may be partitioned or distributed between or among dedicated processors for the immunoassay and clinical chemistry analyzers and a control processor in communication with the dedicated processors. In addition, each of these systems may be implemented as Expert Systems. As noted, the Reflex algorithm may be implemented to provide additional treatment or testing recommendations based on the specific progression of results along a measurement path (also referred to herein as sequence or thread). To further enhance diagnostic efficacy of such an Expert system, the processing systems may also have access to additional patient data (e.g., via a database), such as patient medical history, family history, results from additional tests performed (e.g., electrocardiogram analysis) since admission, etc., and diagnosis and/or treatment recommendations may also account for such information. Of course, such an Expert system may be implemented off-line and/or on a network by a processor which is independent of the analyzers or control processor, and which has access to AMI Reflex method results and other patient data (e.g., coupled to a patient database to which Reflex method testing results are written).

It is further appreciated that a Reflex algorithm for AMI is only one of myriad possible reflex-type algorithms that may be developed for implementation by the foregoing illustrative systems which include both an immunochemistry analyzer and a clinical chemistry analyzer under control of at least one processor implementing a reflex-type algorithm. For example, such a system may be employed to implement reflex-type algorithms that may be developed in order to diagnose other pathologies such as liver disease, lipid risk, or other pathologies where both immunoassays and clinical chemistry assays are used. Further, it is understood that such a system may further integrate additional instrumentation to implement any further testing (e.g., hematology, urinalysis) required by the reflex algorithm.

The following example is meant to illustrate and exemplify various aspects of carrying out the present invention and is not intended to limit the invention.

### EXAMPLE

The reflex algorithm in accordance with the present invention was evaluated and refined during a clinical trial at Hartford Hospital, Hartford, CT, in which it was demonstrated that performance of the algorithm can reduce the number of tests performed on a patient by about 70%, compared with a testing scheme in which all markers were tested regardless of the outcome of previous tests. Other algorithms in the art suffer from the latter type of test scheme, which is more time consuming and costly than the novel reflex algorithm of the present invention.

The experiment comprised measurements of creatine kinase (tCK) activity, its MB isoenzyme (CKMB) as a mass assay, myoglobin, and cardiac troponin I (cTNI) for blood samples collected serially, upon admission and in four hour increments up through twelve hours post-admission, on patients presenting to the emergency room with a chief complaint of chest pain. Analysis of total CK was performed on a Technicon RA-XT whereas all other assays were performed on an Immuno 1 instrument (Bayer Corporation). The diagnostic results based on all such measurements were compared with the results provided by applying the Reflex algorithm of **FIGS. 1A-1F** to the measured data. In the Reflex algorithm, the cut-off (i.e., threshold) levels for Myoglobin, CKMB and Troponin I were used as recommended by the manufacturer of the test (i.e., Bayer in this case), and the cut-off level for t-CK was set at 100 U/L although the manufacturer recommends 130 U/L; this is to help to avoid false positive results during reflex testing. The cut-off for the %RI (Percent Relative Index) was set at 4% For the purpose of this experimental study the data were run through the Reflex algorithm manually. It is noted that development of this illustrative Reflex algorithm considered the cost-effective use of cardiac markers for the emergency department evaluation of patients with chest pain.

Based on the results from the biochemical marker measurements, patients were categorized by the Reflex algorithm into one of eighteen possible endpoints (steps shown as including labels **A-R** in **FIGS. 1A-1F)** in the Reflex algorithm. Diagnoses for: endpoints **A** and **B** would require only the first sample at the time of admission; endpoints **C, D, E** and **F** would require only the first blood sample and an additional blood sample drawn 4 hours post-admission; endpoints **G, H** and **I** would require blood samples drawn up through 8 hours post-admission; endpoints **J, K, L, M** would require blood samples drawn through 12 hours post-admission; endpoints **N, O** and **P** would require blood samples drawn up through 16 hours post-admission; and endpoints **Q** and **R** would require blood samples drawn through 20 hours post-admission.

The distribution of patients with AMI (sample size of n=34), as determined from all biochemical marker measurements, reaching various endpoints according to application of the Reflex algorithm was as follows: **A** = 52.94%; **B** = 11.76%; **C** = 17.64%; **E** = 2.94%; **F** = 11.76%; **I** = 2.94%. All the patients in non-AMI group (n=67) reached endpoint **J**, 12 hours post-admission. Notably, no diagnosis required any samples drawn beyond 12 hours post-admission, which represents the unlikelihood of a patient having a positive myoglobin result and a negative total CK result at 8 hours post admission after demonstrating negative total CK upon admission and four hours later. Nevertheless, provision of such a pathway in the illustrative Reflex algorithm is useful to diagnose detecting the possibility of early AMI beyond four hours post-admission.

The experimental results therefore show that 64.70% of AMI cases were diagnosed using the Reflex algorithm with only the first sample at admission, 32.34% with the first sample at admission and a sample drawn about 4 hours later, and the remaining 2.94% with the first sample at admission as well as samples drawn 4 hours and 8 hours post-admission. For this entire sample of patients (i.e., both AMI and non-AMI). using only the Reflex algorithm for diagnosing myocardial status would have reduced the number of tests from 1616 if all markers were tested to 589. It is noted that this reduction in the number of tests required to diagnose AMI according to the Reflex algorithm is due, in part, to the hierarchical arrangement of tests such that normal results for myoglobin and total CK for the first two samples separated by about 4-6 hours obviates the need for CKMB, and abnormal results for myoglobin and CKMB may be sufficient to indicate presence of acute myocardial infarction without the need to perform cTNI.

The data thus clearly demonstrates that the illustrative Reflex algorithm of **FIGS. 1A-1F** according to the present invention is an efficient tool for proper utilization of cardiac markers in early detection of myocardial damage and can substantially reduce the cost by appropriate utilization of laboratory services.

As may be appreciated from the foregoing description, including the illustrative embodiment, the illustrative variations and modifications, and the example, and as may be further appreciated by practicing the present invention, the present invention provides myriad advantages and attendant advantages. For instance, a Reflex method according to the present invention not only helps determine the appropriate biochemical tests, but also eliminates unnecessary assays. Accordingly, it provides for a cost effective, unambiguous, and early diagnosis of acute myocardial infarctions and a stratification of risk for non-AMI patients, such as those with unstable angina pectoris. Since each step in the Reflex method is determined based solely on the results of a previous assay results, and all possible combinations of laboratory results are covered, no human decision making is required and the Reflex method is thus well suited for ministerial operator implementation, and particularly well suited for programmatic implementation, and more particularly well suited for incorporation into the software of automated diagnostic platforms which do not require any operator intervention. It was also found that eliminating human decision making from test determination resulted in faster and more cost effective diagnosis of AMI.

Although the above description of illustrative embodiments of the invention, and various modifications thereof, provides many specificities, these enabling details should not be construed as limiting the scope of the invention, and it will be readily understood by those persons skilled in the art that the present invention is susceptible to many modifications, adaptations, and equivalent implementations without departing from this scope and without diminishing its attendant advantages. It is therefore intended that the present invention is not limited to the disclosed embodiments but should be defined in accordance with the claims which follow.

## Claims

1. A diagnostic system comprising:
immunoassay instrumentation (42) that allows automatic execution of an immunoassay measurement;
**characterized in that** the diagnostic system further comprises
a clinical chemistry instrumentation (44) that allows automatic execution of a clinical chemistry assay measurement;
a sample handling device (46) coupled between said immunoassay instrumentation and said clinical chemistry instrumentation to allow sharing of samples therebetween; and
a processor (40) coupled to said immunoassay instrumentation (42) and said clinical chemistry instrumentation (44), wherein said processor (40) selectively commands said immunoassay instrumentation (42) and said clinical chemistry instrumentation (44) to execute measurements according to an algorithm, which specifies selection of subsequent tests based on results of previous tests (= reflex algorithm) and which includes at least one immunoassay and at least one clinical chemistry assay.

2. The diagnostic system according to claim 1, wherein said immunoassay instrumentation (42) and said clinical chemistry instrumentation (44) each have a respective local processor in communication with said processor (40), wherein the local processors respectively control execution of measurements specified by said processor (40) on the immunoassay instrumentation (42) and clinical chemistry instrumentation (44).

3. The diagnostic system according to claim 2, wherein said processor (40) communicates with each of said local processors via a network.

4. The diagnostic system according to claim 3, wherein said network is a public or private network.

5. The diagnostic system according to claim 2, wherein said local processors each independently and selectively execute a local program or subroutine to control a sequence of measurements in response to a command from said processor (40).

6. The diagnostic system according to claim 1, wherein said processor (40) selectively suggests and indication of pathology based, at least in part, on results from the measurements executed according to said reflex algorithm.

7. The diagnostic system according to claim 1, further comprising a hematology analyzer coupled to said processor (40), and wherein said measurements include a measurement executed by the hematology analyzer in response to a command from said processor (40).

8. The diagnostic system according to claim 1, further comprising a computer-readable medium accessible to said processor (40) and that store information that represents the reflex algorithm.

9. The diagnostic system according to claim 1, wherein said processor (40) is in communication with said immunoassay instrumentation (42) and said clinical chemistry instrumentation (44) such that said processor (40) receives information concerning outputs from the measurements conducted on the immunoassay instrumentation (42) and on the clinical chemistry instrumentation (44).

10. A computer program embodied on a computer-readable medium, for controlling automatic execution of assay measurements by clinical chemistry assay instrumentation (44) and by immunoassay instrumentation (42) in a diagnostic system with an
immunoassay instrumentation (42) that allows automatic execution of an immunoassay measurement;
a clinical chemistry instrumentation (44) that allows automatic execution of a clinical chemistry assay measurement;
a sample handling device (46) coupled between said immunoassay instrumentation and said clinical chemistry instrumentation to allow sharing of samples therebetween; and
a processor (40) coupled to said immunoassay instrumentation (42) and said clinical chemistry instrumentation (44), wherein said processor (40) selectively commands said immunoassay instrumentation (42) and said clinical chemistry instrumentation (44) to execute measurements according to an algorithm, which specifies selection of subsequent tests based on results of previous tests (= reflex algorithm) and which includes at least one immunoassay and at least one clinical chemistry assay,
comprising:
a first code segment including a sequence of instructions to determine measurements to be executed by the clinical chemistry assay instrumentation (44) and the immunoassay instrumentation (42) according to a stored representation of the reflex algorithm to facilitate diagnostic of a pathology; and
a communication code segment including instructions operative in sending to the immunoassay instrumentation (42) and the clinical chemistry instrumentation (44) commands to execute the determined measurements, and instructions operative in receiving information concerning results from the measurements executed by the immunoassay instrumentation (42) and the clinical chemistry instrumentation (44).

11. The computer program embodied on a computer-readable medium according to claim 10, further comprising a code segment to output an indication of a pathology on the reflex algorithm and on the measurements.

12. The computer program embodied on a computer-readable medium according to claim 10, wherein said first code segment and said communication code segment are executed on a first processor (40), and wherein the immunoassay instrumentation and the clinical chemistry instrumentation each have a respective processor in communication with said first processor (40).

13. A method for selectively executing a sequence of biochemical marker measurement steps, the method comprising the steps of:
providing an immunoassay instrumentation (42),
**characterised in that**,
further a clinical chemistry assay instrumentation (44) is provided; and
an automatic sample handler (46) is coupled between the immunoassay instrumentation (42) and the clinical chemistry assay instrumentation (44); and
samples are shared between the clinical chemistry assay instrumentation (44) and the immunoassay instrumentation (42) by using the automatic sample handler (46); and
biochemical marker measurements including measuring a concentration level or an activity of at least one biochemical marker in a urine, serum, plasma or whole blood sample are conducted; and
a processor (40) is provided for receiving information selectively from the immunoassay (42) and clinical chemistry assay instrumentation (44) concerning outputs from biochemical marker measurements conducted on the immunoassay instrumentation (42) or clinical chemistry instrumentation (44); and
the processor (40) selectively sends commands to the immunoassay instrumentation (42) and the clinical chemistry assay instrumentation (44) to execute a biochemical marker measurement selected by the processor (40) based on a stored representation of an algorithm, which specifies selection of subsequent tests based on results of previous tests (= reflex algorithm), which includes at least one immunoassay and at least one clinical chemistry assay.

14. The method according to claim 13, further comprising the processor executed step of specifying an indication of a pathology according to the stored representation of the reflex algorithm and on information concerning outputs from the biochemical marker measurements.

15. The method according to claim 13, wherein the reflex algorithm logically represents a plurality of sequences of the biochemical marker measurement steps as prescribed by a stored representation of a decision tree, each of the biochemical marker measurement steps including measuring a concentration level or activity of at least one biochemical marker in a urine, serum, plasma or whole blood sample obtained from said individual at a time specified by the decision tree, each sequence of the decision tree beginning with a common first biochemical marker measurement step conducted on a first urine, serum, plasma or whole blood sample obtained from an individual at a first time specified by the decision tree, each of the biochemical marker measurement steps subsequent tot he common first step selectively performed based on results from a precedent biochemical marker measurement step, each sequence terminating in a respective final biochemical marker measurement step conducted on urine, serum, plasma or whole blood sampled from said individual at a time prescribed by the stored representation the decision tree.

16. The method according to claim 15, further comprising the step of providing an indication of a pathology for said individual based on the sequence performed and on the results of the respective final biochemical marker measurement step.

17. The method according to claim 16, wherein said step of providing an indication includes providing the indication based on the results of the biochemical marker measurements in the sequence performed, thereby depending on the specific sequence performed.

18. The method according to claim 15, wherein one or more points along said decision tree indicate a recommended treatment or test other than the biochemical marker measurements within the sequences of the decision tree.

19. The method according to claim 18, wherein the recommended treatment or test other than the biochemical marker measurements is provided subsequent to one or more of said respective final biochemical marker measurement steps.

## Patentansprüche

1. Diagnose-System, umfassend:
eine Immunoassay-Instrumentation (42), mit der die automatische Durchführung einer Immunoassay-Messung ermöglicht wird,
**dadurch gekennzeichnet, dass** das Diagnose-System ferner umfasst:
eine Instrumentation (44) für klinische Chemie, welche eine automatische Durchführung einer Assay-Messung für die klinische Chemie ermöglicht;
eine Proben-Handhabungseinrichtung (46), gekoppelt zwischen der genannten Immunoassay-Instrumentation und der genannten Instrumentation für die klinische Chemie, um es zu ermöglichen, dass Proben dazwischen aufgeteilt werden; sowie
einen Prozessor (40), gekoppelt an die genannte Immunoassay-Instrumentation (42) und an die genannte Instrumentation (44) für die klinische Chemie, worin der genannte Prozessor (40) selektiv der genannten Immunoassay-Instrumentation (42) und der genannten Instrumentation (44) für die klinische Chemie Befehle erteilt, Messungen gemäß einem Algorithmus durchzuführen, welcher die Auswahl anschließender Tests auf der Grundlage von Ergebnissen vorheriger Tests (= Reflex-Algorithmus) spezifiziert und mindestens einen Immunoassay und mindestens einen Assay für die klinische Chemie einschließt.

2. Diagnose-System gemäß Anspruch 1, worin jede der genannten Immunoassay-Instrumentation (42) und der genannten Instrumentation (44) für die klinische Chemie einen jeweiligen örtlichen Prozessor im Zusammenschluss mit dem genannten Prozessor (40) aufweist, worin die örtlichen Prozessoren jeweils die Durchführung von Messungen steuern, die vom genannten Prozessor (40) auf der Immunoassay-Instrumentation (42) und der Instrumentation (44) für die klinische Chemie spezifiziert werden.

3. Diagnose-System gemäß Anspruch 2, worin der genannte Prozessor (40) mit jedem der genannten örtlichen Prozessoren über ein Netzwerk in Verbindung steht.

4. Diagnose-System gemäß Anspruch 3, worin das genannte Netzwerk ein öffentliches oder privates Netzwerk ist.

5. Diagnose-System gemäß Anspruch 2, worin die genannten örtlichen Prozessoren jeder unabhängig und selektiv ein örtliches Programm oder eine entsprechende Subroutine vollziehen, um eine Abfolge von Messungen in Reaktion auf einen Befehl aus dem genannten Prozessor (40) zu steuern.

6. Diagnose-System gemäß Anspruch 1, worin der genante Prozessor (40) selektiv eine Indikation einer Pathologie vorschlägt, welche zumindest teilweise auf Ergebnisse aus den gemäß dem genannten Reflex-Algorithmus durchgeführten Messungen bezogen ist.

7. Diagnose-System gemäß Anspruch 1, ferner umfassend ein Hämatologie-Analysegerät, gekoppelt an den genannten Prozessor (40), worin die genannten Messungen eine Messung einschließen, die vom Hämatologie-Analysegerät in Reaktion auf einen Befehl aus dem genannten Prozessor (40) durchgeführt wird.

8. Diagnose-System gemäß Anspruch 1, ferner umfassend ein Computer-lesbares Medium, das für den genannten Prozessor (40) zugänglich ist und Information speichert, die den Reflex-Algorithmus darstellen.

9. Diagnose-System gemäß Anspruch 1, worin der genannte Prozessor (40) in Verbindung mit der genannten Immunoassay-Instrumentation (42) und der genannten Instrumentation (44) für die klinische Chemie steht, so dass der genannte Prozessor (40) Information empfängt, die Ausgaben aus den Messungen betrifft, die an der Immunoassay-Instrumentation (42) und an der Instrumentation (44) für die klinische Chemie durchgeführt werden.

10. Computer-Programm, ausgestaltet an einem Computerlesbaren Medium, zur Steuerung einer automatischen Durchführung von Assay-Messungen durch eine Assay-Instrumentation (44) für klinische Chemie und eine Immunoassay-Instrumentation (42) in einem Diagnose-System mit einer
Immunoassay-Instrumentation (44), die eine automatische Durchführung einer Immunoassay-Messung ermöglicht,
einer Instrumentation (44) für die klinische Chemie, die eine automatische Durchführung einer Assay-Messung für die klinische Chemie ermöglicht,
einer Proben-Handhabungseinrichtung (46), gekoppelt zwischen der genannten Immunoassay-Instrumentation und der genannten Instrumentation für die klinische Chemie, um es zu ermöglichen, dass Proben dazwischen verteilt werden, sowie mit
einem Prozessor (40), gekoppelt an die genannte Immunoassay-Instrumentation (42) und an die genannte Instrumentation (44) für die klinische Chemie, worin der genannte Prozessor (40) selektiv der genannten Immunoassay-Instrumentation (42) und der genannten Instrumentation (44) für die klinische Chemie Befehle erteilt, um Messungen gemäß einem Algorithmus durchzuführen, welcher die Auswahl anschließender Tests auf der Grundlage von Ergebnisse vorheriger Tests spezifiziert (= Reflex-Algorithmus) und mindestens einen Immunoassay und mindestens einen Assay für die klinische Chemie einschließt,
umfassend:
ein erstes Code-Segment, welches eine Abfolge von Anweisungen einschließt, um Messungen zu bestimmen, die von der Assay-Instrumentation (44) für die klinische Chemie und von der Immunoassay-Instrumentation (42) gemäß einer gespeicherten Darstellung des Reflex-Algorithmus durchzuführen sind, um die Diagnostik einer Pathologie zu erleichtern, sowie
ein Kommunikations-Code-Segment, das Anweisungen, die dazu dienen, an die Immunoassay-Instrumentation (42) und an die Instrumentation (44) für die klinische Chemie Befehle zur Durchführung der bestimmten Messungen zu senden, und Anweisungen einschließt, die dazu dienen, Information zu empfangen, die Ergebnisse aus den Messungen betrifft, die von der Immunoassay-Instrumentation (42) und der Instrumentation (44) für die klinische Chemie durchgeführt werden.

11. Computer-Programm, ausgestaltet an einem Computerlesbaren Medium, gemäß Anspruch 10, ferner umfassend ein Code-Segment zur Ausgabe einer Indikation einer Pathologie auf dem Reflex-Algorithmus und auf den Messungen.

12. Computer-Programm, ausgestaltet an einem Computerlesbaren Medium, gemäß Anspruch 10, worin das genannte erste Code-Segment und das genannte Kommunikations-Code-Segment an einem ersten Prozessor (40) vollzogen werden, und worin die Immunoassay-Instrumentation und die Instrumentation für die klinische Chemie jede einen jeweiligen Prozessor in Zusammenschluss mit dem genannten ersten Prozessor (40) aufweisen.

13. Verfahren zum selektiven Vollzug einer Abfolge biochemischer Marker-Messtufen, wobei das Verfahren die Stufen umfasst, in denen man:
eine Immunoassay-Instrumentation (42) bereitstellt,
**dadurch gekennzeichnet, dass**
ferner eine Assay-Instrumentation (44) für klinische Chemie bereitgestellt,
eine automatische Proben-Handhabungseinrichtung (46) zwischen die Immunoassay-Instrumentation (42) und die Instrumentation (44) für die klinische Chemie gekoppelt,
Proben zwischen der Assay-Instrumentation (44) für die klinische Chemie und der Immunoassay-Instrumentation (42) durch Anwendung der automatischen Proben-Handhabungseinrichtung (46) verteilt,
biochemische Marker-Messungen, einschließlich der Messung einer Konzentrationsgehaltsmenge oder einer Aktivität von mindestens einem biochemischen Marker, in einer Urin-, Serum-, Plasma- oder Gesamtblutprobe durchgeführt,
ein Prozessor (40) bereitgestellt werden, um Information selektiv aus dem Immunoassay (42) und der Assay-Instrumentation (44) für die klinische Chemie zu empfangen, welche Ausgaben aus biochemischen Marker-Messungen betrifft, die an der Immunoassay-Instrumentation (42) oder der Instrumentation (44) für die klinische Chemie durchgeführt werden, und dass der Prozessor (40) selektiv Befehle an die Immunoassay-Sedimen-tation (42) und die Instrumentation (44) für die klinische Chemie sendet, um eine biochemische Marker-Messung durchzuführen, die von Prozessor (40) auf der Grundlage einer gespeicherten Darstellung eines Algorithmus ausgewählt wird, welcher die Auswahl anschließender Tests auf der Grundlage von Ergebnissen vorheriger Tests spezifiziert (= Reflex-Algorithmus), welcher mindestens einen Immunoassay und mindestens einen Assay für klinische Chemie einschließt.

14. Verfahren gemäß Anspruch 13, welches ferner die vom Prozessor durchgeführte Stufe umfasst, in der die Indikation einer Pathologie gemäß der gespeicherten Darstellung des Reflex-Algorithmus und auf einer Information spezifiziert wird, die Ausgaben aus den biochemischen Marker-Messungen betrifft.

15. Verfahren gemäß Anspruch 13, worin der Reflex-Algorithmus in logischer Weise eine Vielzahl von Abfolgen der biochemischen Marker-Messtufen gemäß Vorschrift durch eine gespeicherte Darstellung eines Entscheidungsbaums darstellt, wobei jede der biochemischen Marker-Messtufen die Messung einer Konzentrationsgehaltsmenge oder einer Aktivität von zumindest einem biochemischen Marker in einer Urin-, Serum-, Plasma- oder Gesamtblutprobe einschließt, die von einer genannten Einzelperson zu einem durch den Entscheidungsbaum spezifizierten Zeitpunkt erhalten wird, wobei jede Abfolge des Entscheidungsbaums mit einer gemeinsamen ersten biochemischen Marker-Messtufe beginnt, die an einer ersten Urin-, Serum-, Plasma- oder Gesamtblut-Probe durchgeführt wird, die von einer Einzelperson zu einem durch den Entscheidungsbaum spezifizierten ersten Zeitpunkt erhalten wird, wobei jede der biochemischen Marker-Messtufen im Anschluss an die gemeinsame erste Stufe selektiv auf der Grundlage von Ergebnissen aus einer vorhergehenden biochemischen Marker-Messtufe durchgeführt wird, und wobei jede Abfolge in einer jeweiligen endgültigen biochemischen Marker-Messtufe endet, die mit Urin, Serum, Plasma oder Gesamtblut durchgeführt wird, welche als Probe von der genannten Einzelperson zu einem Zeitpunkt gezogen wird, der durch die gespeicherte Darstellung im Entscheidungsbaum vorgeschrieben ist.

16. Verfahren gemäß Anspruch 15, ferner umfassend die Stufe, in der die Indikation einer Pathologie für die genannte Einzelperson auf der Grundlage der durchgeführten Abfolge und der Ergebnisse der jeweiligen endgültigen biochemischen Marker-Messstufe angegeben wird.

17. Verfahren gemäß Anspruch 16, worin die genannte Stufe zur Angabe der Indikation einschließt, dass die Indikation auf der Grundlage der Ergebnisse der biochemischen Marker-Messungen in der durchgeführten Abfolge angegeben wird und dadurch von der durchgeführten spezifischen Abfolge abhängt.

18. Verfahren gemäß Anspruch 15, worin einer oder mehrere Punkte entlang des genannten Entscheidungsbaums eine empfohlene Behandlung oder einen Test indizieren, die sich von den biochemischen Marker-Messungen innerhalb der Abfolgen des Entscheidungsbaums unterscheiden.

19. Verfahren gemäß Anspruch 18, worin die empfohlene Behandlung oder der Test, die sich von den biochemischen Marker-Messungen unterscheiden, im Anschluss an eine oder mehrere der genannten jeweiligen endgültigen biochemischen Marker-Messtufen angegeben werden.

## Revendications

1. Système de diagnostic comprenant :
un appareillage de dosage immunologique (42) qui permet l'exécution automatique d'une mesure de dosage immunologique ;
**caractérisé en ce que** le système de diagnostic comprend en outre
un appareillage de chimie clinique (44) qui permet l'exécution automatique d'une mesure de dosage de chimie clinique ;
un dispositif de manipulation d'échantillon (46) couplé entre ledit appareillage de dosage immunologique et ledit appareillage de chimie clinique pour permettre le partage d'échantillons entre eux ; et
un processeur (40) couplé audit appareillage de dosage immunologique (42) et audit appareillage de chimie clinique (44), dans lesquels ledit processeur (40) commande de manière sélective ledit appareillage de dosage immunologique (42) et ledit appareillage de chimie clinique (44) pour exécuter des mesures selon un algorithme qui spécifie une sélection d'essais postérieurs en fonction des résultats d'essais précédents (= algorithme réflexe) et qui inclut au moins un dosage immunologique et au moins un dosage de chimie clinique.

2. Système de diagnostic selon la revendication 1, dans lequel ledit appareillage de dosage immunologique (42) et ledit appareillage de chimie clinique (44) ont chacun un processeur local respectif en communication avec ledit processeur (40), dans lequel les processeurs locaux contrôlent respectivement l'exécution de mesures spécifiées par ledit processeur (40) sur l'appareillage de dosage immunologique (42) et l'appareillage de chimie clinique (44).

3. Système de diagnostic selon la revendication 2, dans lequel ledit processeur (40) communique avec chacun desdits processeurs locaux par l'intermédiaire d'un réseau.

4. Système de diagnostic selon la revendication 3, dans lequel ledit réseau est un réseau public ou privé.

5. Système de diagnostic selon la revendication 2, dans lequel lesdits processeurs locaux exécutent chacun de manière indépendante et sélective un programme local ou un sous-programme pour contrôler une suite de mesures en réponse à une commande dudit processeur (40).

6. Système de diagnostic selon la revendication 1, dans lequel ledit processeur (40) suggère de manière sélective une indication de pathologie fondée, au moins en partie, sur des résultats des mesures exécutées selon ledit algorithme réflexe.

7. Système de diagnostic selon la revendication 1, comprenant en outre un analyseur d'hématologie couplé audit processeur (40) et dans lequel lesdites mesures incluent une mesure exécutée par l'analyseur d'hématologie en réponse à une commande dudit processeur (40).

8. Système de diagnostic selon la revendication 1, comprenant en outre un moyen lisible par ordinateur accessible audit processeur (40) et qui stocke des informations qui représentent l'algorithme réflexe.

9. Système de diagnostic selon la revendication 1, dans lequel ledit processeur (40) est en communication avec ledit appareillage de dosage immunologique (42) et ledit appareillage de chimie clinique (44) de sorte que ledit processeur (40) reçoit des informations concernant des résultats des mesures effectuées sur l'appareillage de dosage immunologique (42) et sur l'appareillage de chimie clinique (44).

10. Programme d'ordinateur réalisé sur un moyen lisible par ordinateur, pour commander l'exécution automatique de mesures de dosage par un appareillage de dosage de chimie clinique (44) et par un appareillage de dosage immunologique (42) dans un système de diagnostic avec
un appareillage de dosage immunologique (42) qui permet l'exécution automatique d'une mesure de dosage immunologique ;
un appareillage de chimie clinique (44) qui permet l'exécution automatique d'une mesure de dosage de chimie clinique ;
un dispositif de manipulation d'échantillon (46) couplé entre ledit appareillage de dosage immunologique et ledit appareillage de chimie clinique pour permettre le partage d'échantillons entre eux ; et
un processeur (40) couplé audit appareillage de dosage immunologique (42) et audit appareillage de chimie clinique (44), dans lequel ledit processeur (40) commande de manière sélective ledit appareillage de dosage immunologique (42) et ledit appareillage de chimie clinique (44) pour exécuter des mesures selon un algorithme qui spécifie une sélection d'essais en fonction des résultats d'essais postérieurs précédents (= algorithme réflexe) et qui inclut au moins un dosage immunologique et au moins un dosage de chimie clinique,
comprenant :
un premier segment de codage incluant une suite d'instructions pour déterminer des mesures à exécuter par l'appareillage de dosage de chimie clinique (44) et l'appareillage de dosage immunologique (42) selon une représentation mémorisée de l'algorithme réflexe pour faciliter le diagnostic d'une pathologie ; et
un segment de codage de communication incluant des instructions utilisables dans l'envoi à l'appareillage de dosage immunologique (42) et à l'appareillage de chimie clinique (44) des commandes pour exécuter les mesures déterminées et des instructions utilisables dans la réception d'informations concernant les résultats des mesures exécutées par l'appareillage de dosage immunologique (42) et l'appareillage de chimie clinique (44).

11. Programme d'ordinateur réalisé sur un moyen lisible par ordinateur selon la revendication 10, comprenant en outre un segment de codage pour émettre une indication d'une pathologie sur l'algorithme réflexe et sur les mesures.

12. Programme d'ordinateur réalisé sur un moyen lisible par ordinateur selon la revendication 10, dans lequel ledit premier segment de codage et ledit segment de codage de communication sont exécutés sur un premier processeur (40) et dans lequel l'appareillage de dosage immunologique et l'appareillage de chimie clinique ont chacun un processeur respectif en communication avec ledit premier processeur (40).

13. Procédé pour exécuter de manière sélective une suite d'étapes de mesure de marqueur biochimique, le procédé comprenant les étapes qui consistent à :
procurer un appareillage de dosage immunologique (42), **caractérisé en ce que**
un autre appareillage de dosage de chimie clinique (44) est proposé ; et
un dispositif de manipulation d'échantillon automatique. (46) est couplé entre l'appareillage de dosage immunologique (42) et l'appareillage de dosage de chimie clinique (44) ; et
des échantillons sont partagés entre l'appareillage de dosage de chimie clinique (44) et l'appareillage de dosage immunologique (42) en utilisant le dispositif de manipulation d'échantillon automatique (46) ; et
des mesures de marqueur biochimique incluant le mesurage d'un niveau de concentration ou d'une activité d'au moins un marqueur biochimique dans un échantillon d'urine, de sérum, de plasma ou de sang total sont effectuées ; et
un processeur (40) est proposé pour recevoir des informations de manière sélective de l'appareillage de dosage immunologique (42) et de l'appareillage de dosage de chimie clinique (44) concernant des résultats de mesures de marqueur biochimique effectuées sur l'appareillage de dosage immunologique (42) ou l'appareillage de chimie clinique (44) ; et
le processeur (40) envoie de manière sélective des commandes à l'appareillage de dosage immunologique (42) et à l'appareillage de dosage de chimique clinique (44) pour exécuter une mesure de marqueur biochimique sélectionnée par le processeur (40) en fonction d'une représentation mémorisée d'un algorithme qui spécifie une sélection d'essais postérieurs en fonction des résultats d'essais précédents (= algorithme réflexe), qui inclut au moins un dosage immunologique et au moins un dosage de chimie clinique.

14. Procédé selon la revendication 13, comprenant en outre l'étape exécutée par le processeur qui consiste à spécifier une indication d'une pathologie selon la représentation mémorisée de l'algorithme réflexe et des informations concernant des résultats de mesures de marqueur biochimique.

15. Procédé selon la revendication 13, dans lequel l'algorithme réflexe représente logiquement une pluralité de suites d'étapes de mesure de marqueur biochimique telles que prescrites par une représentation mémorisée d'un arbre de décision, chacune des étapes de mesure de marqueur biochimique incluant un mesurage d'un niveau de concentration ou d'une activité d'au moins un marqueur biochimique dans un échantillon d'urine, de sérum, de plasma ou de sang total obtenu dudit individu à un moment spécifié par l'arbre de décision, chaque suite de l'arbre de décision commençant par une première étape de mesure de marqueur biochimique commune effectuée sur un premier échantillon d'urine, de sérum, de plasma ou de sang total obtenu d'un individu à un premier moment spécifié par l'arbre de décision, chacune des étapes de mesure de marqueur biochimique étant postérieure à la première étape commune exécutée de manière sélective en fonction des résultats d'une étape de mesure de marqueur biochimique précédente, chaque suite se terminant par une étape de mesure de marqueur biochimique finale respective effectuée sur des échantillons d'urine, de sérum, de plasma ou de sang total prélevés sur ledit individu à un moment prescrit par la représentation mémorisée de l'arbre de décision.

16. Procédé selon la revendication 15, comprenant en outre l'étape qui consiste à proposer une indication d'une pathologie pour ledit individu en fonction de la suite exécutée et des résultats de l'étape de mesure de marqueur biochimique finale respective.

17. Procédé selon la revendication 16, dans lequel ladite étape qui consiste à proposer une indication inclut le fait de proposer l'indication en fonction des résultats des mesures du marqueur biochimique dans la suite exécutée, dépendant par ce moyen de la suite spécifique exécutée.

18. Procédé selon la revendication 15, dans lequel un point ou plus le long dudit arbre de décision indique un traitement ou essai recommandé autre que les mesures de marqueur biochimique dans les limites des suites de l'arbre de décision.

19. Procédé selon la revendication 18, dans lequel le traitement ou l'essai recommandé autre que les mesures de marqueur biochimique est -proposé postérieurement à une étape ou plus de mesures de marqueur biochimique finales respectives.
